Europäisches Patentamt
European Patent Office
Office européen des brevets

(11) Publication number: **0 416 581 A1**

(12) **EUROPEAN PATENT APPLICATION**

(21) Application number: **90117085.2**

(22) Date of filing: **05.09.90**

(51) Int. Cl.[5]: **C07D 211/58**, C07D 211/94, C07D 401/12, C07D 405/12, C07D 409/12, A61K 31/445

(30) Priority: **05.09.89 US 402951**
**05.09.89 US 403205**
**27.08.90 US 571911**

(43) Date of publication of application:
**13.03.91 Bulletin 91/11**

(84) Designated Contracting States:
**AT BE CH DE DK ES FR GB GR IT LI LU NL SE**

(71) Applicant: **G.D. Searle & Co.**
**P.O. Box 5110**
**Chicago Illinois 60680(US)**

(72) Inventor: **Desai, Bipinchandra Nanubhai**
**200 Annapolis Drive**
**Vernon Hills, Illinois 60061(US)**
Inventor: **Fowler, Kerry Wallace**
**747 North 66th Street**
**Seattle, Washington 98103(US)**
Inventor: **Moormann, Allan Edward**
**9450 Latrobe**
**Skokie, Illinois 60077(US)**
Inventor: **Russell, Mark Andrew**
**4629 Main Street**
**Skokie, Illinois 60076(US)**

(74) Representative: **Beil, Hans Chr., Dr. et al**
**Beil, Wolff und Beil, Rechtsanwälte**
**Adelonstrasse 58 Postfach 80 01 40**
**W-6230 Frankfurt am Main 80(DE)**

(54) **Substituted N-benzylpiperidine amides.**

(57) The present invention encompasses substituted N-benzylpiperidine amides of formula I

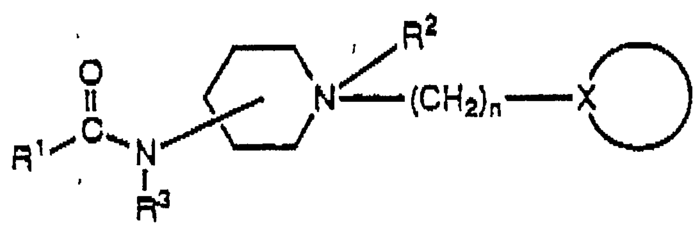

I

with all the substituents $R^1$, $R^2$, $R^3$, n and X as defined in the description as well as pharmaceutical compositions thereof.
These novel compounds are pharmacologically useful in the treatment of cardiac arrhythmias.

EP 0 416 581 A1

# SUBSTITUTED N-BENZYLPIPERIDINE AMIDES

## Background of the Invention

The present invention provides novel compounds, novel compositions, methods of their use and methods of their manufacture, such compounds pharmacologically useful in the treatment of cardiac arrhythmias. More specifically, the compounds of the present invention are Class III antiarrhythmic agents which, by effectively prolonging repolarization of a cardiac cell action potential, can be used effectively to treat certain cardiac arrhythmias.

Antiarrhythmic drugs have been grouped together according to the pattern of electrophysiological effects that they produce and/or their presumed mechanisms of action. Thus, Class I antiarrhythmic agents are characterized by being sodium channel blockers, Class II antiarrhythmic agents are beta-adrenergic blockers, Class III antiarrhythmic agents prolong repolarization, and Class IV antiarrhythmic agents are calcium channel blockers.

Currently, there are very few Class III antiarrhythmic agents available for theraputic use. Among them is bretylium. Bretylium's usefulness is limited, however, and currently its theraputic use is reserved for life-threatening ventricular arrhythmias that are refractory to other therapy. Thus, bretylium's use is generally confined to intensive care units. It is an object of this invention to provide Class III antiarrhythmic agents of broader theraputic use than existing Class III antiarrhythmic agents.

## Summary of the Invention

The invention relates to novel compounds of the general formula I:

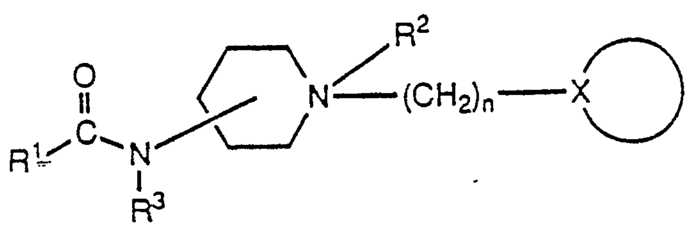

I

and the pharmaceutically acceptable non-toxic salts thereof, and the hydrated forms thereof, wherein $R^1$ is alkenyl or alkynyl of from one to ten carbon atoms; substituted or unsubstituted aralkenyl or aralkynyl of from one to ten carbon atoms and wherein said aryl substituent is one or more of alkoxy, nitro, halogen or alkylsulfonamide at any position with alkoxy or alkylsulfonamide of one to ten carbon atoms; arylamino; aryl fused to substituted or unsubstituted cycloalkyl of from three to eight carbon atoms wherein said cycloalklyl substituent can be halogen; arylcycloalkyl wherein cycloalkyl is from three to eight carbon atoms; substituted or unsubstituted benzofuranyl wherein said benzofuranyl substituent can be one or more of halogen, amino, nitro or alkoxy of from one to ten carbon atoms or alkylsulfonamido wherein the alkyl portion is 1 to 4 carbon atoms at any position; substituted or unsubstituted benzopyran wherein said substituent can be keto; thiophene; benzothiophene; substituted or unsubstituted indene or isoindene wherein said substituent is alkyl of one to ten carbon atoms; or substituted or unsubstituted unsaturated cycloheteroalkenyl wherein the cyclohetero ring consists of 4 to 6 carbon atoms wherein one or two of the said carbon atoms are replaced by nitrogen or oxygen and the substituents are selected from the group consisting of alkyl of from one to ten carbon atoms, halogen, amino, alkoxy of from one to ten carbon atoms or alkylcarboxylate wherein the alkyl is 1 to 6 carbon atoms; substituted or unsubstituted unsaturated cycloheteroalkynyl wherein the cyclohetero ring consists of 4 to 6 carbon atoms wherein one or two of the

said carbon atoms are replaced by nitrogen or oxygen and the substituents are the same as those already described for the unsaturated cycloheteroalkenyl; with the proviso that when $R^2$ is alkyl of 1 to 10 carbon atoms or oxygen $R^1$ can also be aryl or substituted aryl wherein said aryl substituent is one or more of alkyl of from one to ten carbon atoms, halogen, amino or alkoxy of from one to ten carbon atoms.

n is an integer of from one to ten;

$R^2$ is unsubstituted or is alkyl of from one to ten carbon atoms or oxygen that is present as an N-oxide; $R^3$ is hydrogen, carboxyalkyl of from one to ten carbon atoms or alkoxycarbonylalkyl of from one to ten carbon atoms;

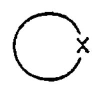

is hydrogen; pyridinyl; cycloalkyl of three to eight carbon atoms or hydroxy substituted cycloalkyl of three to eight carbon atoms; furanyl; or unsubstituted or substituted phenyl wherein said phenyl substituent is one or more of alkyl, halogen substituted alkyl of one to ten carbon atoms, alkoxy from one to ten carbon atoms, nitro, amino, mono cr dialkylamine, acetyl amine, acetylamide, halogen, alkylsulfonamido wherein the alkyl portion is 1 to 4 carbon atoms, alkoxy itself substituted by halogen substituted phenyl or aryalkoxy of from one to ten carbon atoms in length wherein aryl may be substituted by halogen or alkyl of one to ten carbon atoms in length.

The compounds and pharmaceutical compositions thereof are useful in the antiarrhythmic methods of the invention. The invention further provides dosage unit forms adapted for oral, topical and parenteral administration. Also provided for in this invention are the pharmaceutically acceptable salts of the compounds.

## Detailed Description of the Invention

As used herein, the term "alkyl" shall mean straight or branched chain carbon-carbon linkages of from one to ten carbon atoms. "Alkenyl" shall have the same meaning, except that one or more double bonds may be present therein. "Alkynyl" shall have the same meaning, except that one or more triple bonds may be present therein.

"Alkoxy" shall include alkyl, alkenyl and alkynyl, as defined above, substituted by an epoxide oxygen.

"Aralkyl" shall include alkyl, alkenyl and alkynyl, as defined above, substituted by an aryl group, which is defined below.

As used herein, the expression "aryl" is defined as phenyl. The term "substituted aryl" shall include phenyl substituted by alkyl of one to ten carbon atoms. The term "alkyloxyaryl" is defined to include alkyl of one to ten carbon atoms and aryl which may be unsubstituted phenyl or phenyl substituted by alkyl of one to ten carbon atoms. The term "cycloalkyl" is defined to include cyclic alkyl carbon-carbon linkages of three to eight carbon atoms.

The term "benzodioxole" is defined to mean the substituent of the formula

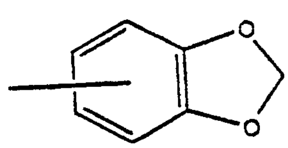

"Halogen" shall include fluorine, chlorine, bromine or iodine.

The term "alkylsulfonamido" shall mean a radical of the formula

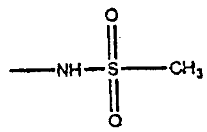

with the understanding that the alkyl portion of the radial may be expanded to 4 carbon atoms.

The term "cardiac arrhythmia" is defined to mean any variation from the normal rhythm of the heartbeat, including, without limitation, sinus arrhythmia, premature heartbeat, heartblock, fibrillation, flutter, pulsus alternans, tachycardia, paroxysmal tachycardia and premature ventricular contractions.

The term "repolarization of cardiac cells" is defined as those phases of a cardiac action potential during which time a depolarized cardiac cell is reverting to normal pre-polarization transmembrane voltage.

The term "pharmaceutically acceptable salts" refers to non-toxic salts of the compounds of this invention which are generally prepared by reacting the free base with a suitable organic or inorganic acid. Representative salts include the hydrochloride, hydroiodic, hydrobromide, sulfate, bisulfate, acetate, oxalate, valerate, oleate, palmitate, stearate, laurate, borate, benzoate, lactate, phosphate, tosylate, citrate, maleate, fumarate, succinate, tartrate, napsylate, clavulanate, methanepercxoate and the like salts.

Compounds of the invention can be prepared readily according to the following reaction scheme or modifications thereof using readily available starting materials, reagents and conventional synthesis procedures. In these reactions, it is also possible to make use of variants which are in themselves known, but are not mentioned in greater detail. $R^1$, $R^2$, $R^3$ and

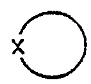

are as defined above. Y is any suitable leaving group, such as halogen, mesylate or tosylate. COZ represents a suitable acylating agent such as a carboxylic acid chloride, a carboxylic acid activated as the mixed anydride, or the carboxylic ester activated by alkylaluminum reagents.

Scheme 1

0121G

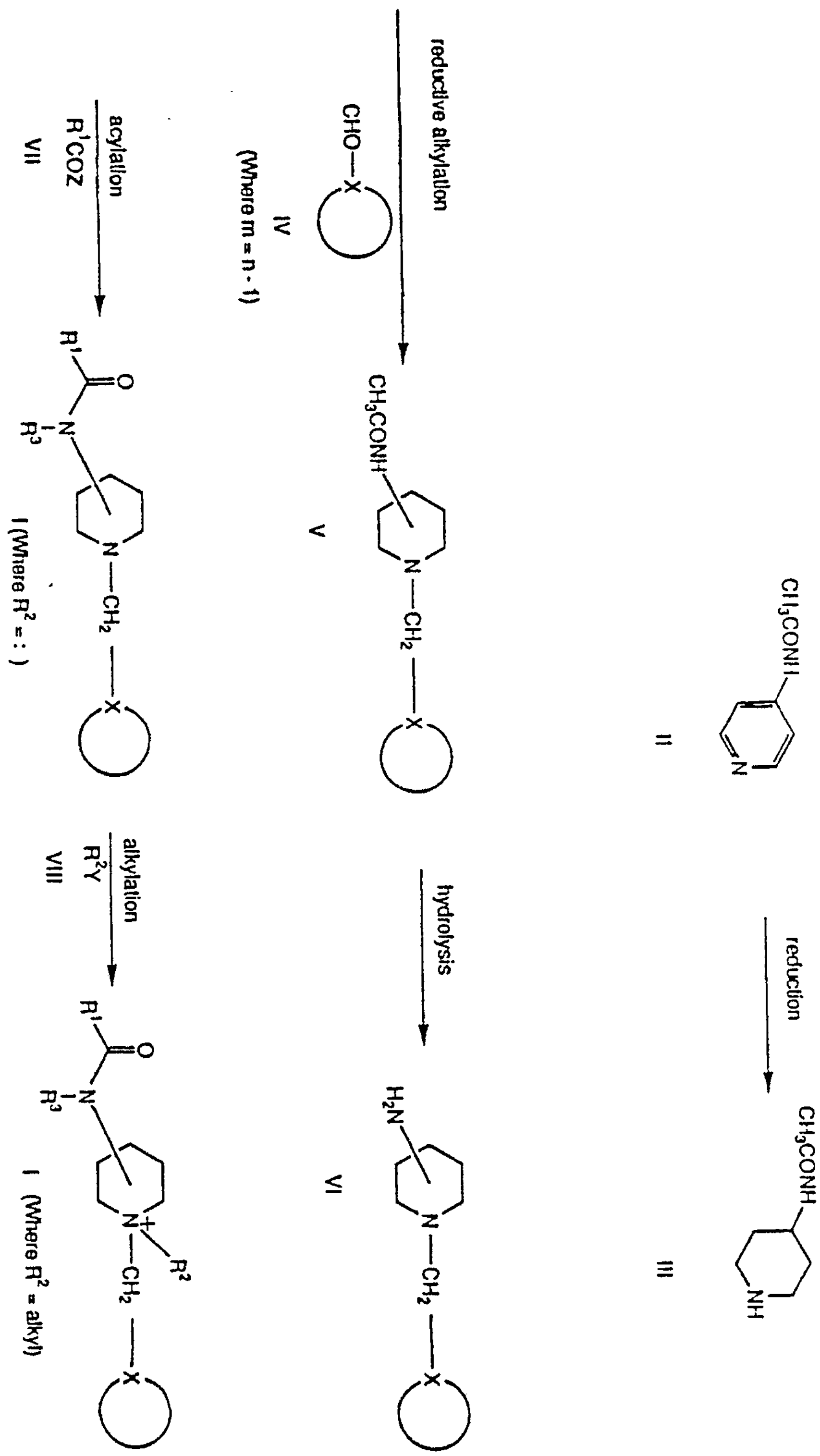

Scheme II

0121G

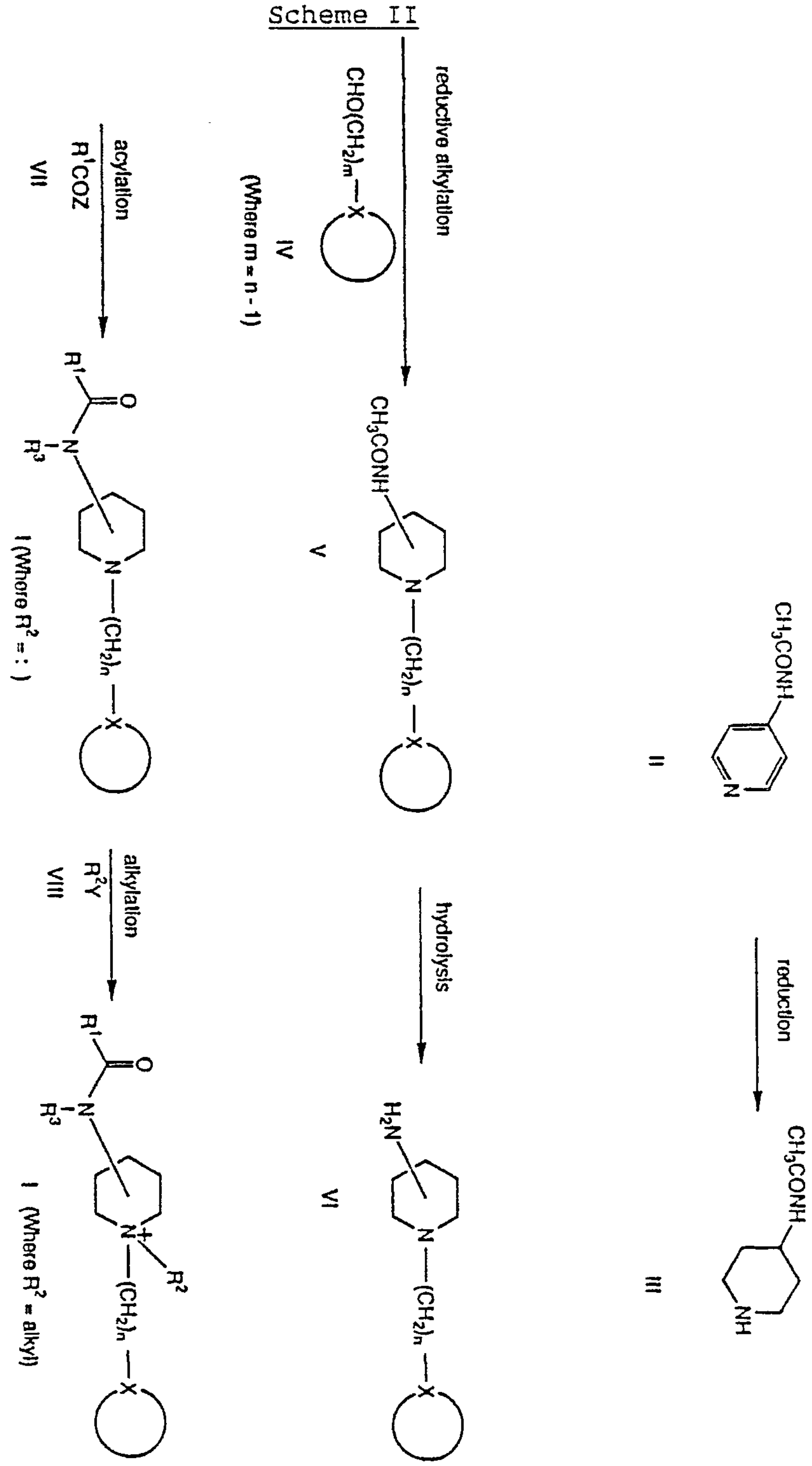


Reduction of 4-acetamidopyridine Formula II affords 4-acetamido-piperidine Formula III. A method for the preparation of 4-acetamidopiperidine III involves the reduction of 4-acylamino N-benzyl pyridinium compounds by alkali metal hydrides or catalytic hydrogenation of the aromatic ring with debenzylation as described in EP 1,537,867 (G.O. Weston) and EP 1,345,872 (J.L. Archibald and J.F. Cavalla) the disclosures of which are incorporated herein by reference. Preferred reduction conditions employ a ruthenium on carbon catalyst in a solvent such as alcohol, tetra hydrofuran, (THF), or acetic acid under an atmosphere of hydrogen. Subsequent reductive alkylation of the piperidine Formula III with aldehydes Formula IV provides the N-alkylated intermediates Formula V. Preferred conditions employ Pt/C catalyst in an inert solvent such

as alcohol, THF, or acetic acid under an atmosphere of hydrogen. Alternative preferred conditions employ borane-pyridine complex as the reducing agent at room temperature in alcohol, acetic acid or methylene chloride. Hydrolysis of the amide bond of acetamides Formula V provides amine intermediates Formula VI. Although hydrolysis may be effected in acid or base, the preferred method employs hydrolysis in 1.2 M HCl at 100° C. Alternative preferred acylating conditions leading to amides I ($R^2$ = lone pair) employ COZ, which can be a carboxylic acid chloride, a carboxylic acid activated as the mixed anhydride, or the carboxylic ester activated by alkylaluminum reagents.

The intermediates Formula I are subsequently converted to the quaternary salts Formula I (where $R^2$ is not an unshared valence bond) by N-alkylating reagents $R^2X$ Formula VIII (where X is a suitable leaving group such as halogen, mesylate, or tosylate) in an inert solvent. Preferred alkylation conditions employ acetonitrile as the solvent at room temperature.

The compounds of the present invention can be administered in such oral dosage forms as tablets, capsules, pills, powders, granules, elixers, tinctures, suspensions, syrups and emulsions. Likewise, it can also be administered in intravenous, intraperitoneal, subcutaneous or intramuscular form, all using forms known to those of ordinary skill in the pharmaceutical arts. In general, the preferred form of administration is oral. An effective but non-toxic amount of the compound is employed in the treatment of arrhythmias of the heart. The dosage regimen utilizing the compound of the present invention is selected in accordance with a variety of factors including the type, species, age, weight, sex and medical condition of the patient; with the severity of the condition to be treated; the route of administration; the renal and hepatic function of the patient; and the particular compound employed or salt thereof. An ordinarily skilled veterinarian or physician can readily determine and prescribe the effective amount of the drug required to prevent, treat or arrest the progress of the condition.

Oral dosages of the compounds of the present invention, when used for the indicated cardiac effects, will range between about 0.1mg per kilogram of body weight per day (mg/kg/day) to about 1000 mg/kg/day and preferably 1.0 to 100 mg/kg/day. Advantageously, the compounds of the present invention can be administered in a single daily dose or the total daily dosage can be administered in divided doses of two, three or four times daily.

In the pharmaceutical compositions and methods of the present invention, the compounds described in detail below will form the active ingredient that will typically be administered in admixture with suitable pharmaceutical diluents, excipients or carriers (collectively referred to herein as "carrier" materials) suitably selected with respect to the intended form of administration, that is, oral tablets, capsules, elixers, syrups and the like, and consistent with conventional pharmaceutical practices.

For instance, for oral administration in the form of tablets or capsules, the active drug component can be combined with an oral non-toxic pharmaceutically acceptable inert carrier such as lactose, starch, sucrose, glucose, methylcellulose, magnesium stearate, dicalcium phosphate, calcium sulfate, mannitol, sorbitol and the like; for oral administration in liquid form, the active drug components can be combined with any oral non-toxic pharmaceutically acceptable inert carrier such as ethanol, glycerol, water, and the like. In the case of oral administration and in liquid form, suitable flavoring carriers can be added such as cherry syrup and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol and various waxes. Lubricants for use in these dosage forms include magnesium stearate, sodium benzoate, sodium acetate, sodium stearate, sodium chloride, sodium oleate and the like. Disintegrators include, without limitation, starch, methylcellulose, agar, bentonite, xanthan gum and the like.

The compounds of this invention can also be administered by intravenous route in doses ranging from 0.01 to 10 mg/kg/day.

Furthermore, it is also contemplated that the invention can be administered in an intranasal form topically via the use of suitable intranasal vehicles, or via transdermal routes, using those forms of transdermal skin patches well known to those of ordinary skill in that art. In the case of transdermal skin patch administration, daily dosage is continuous via the transdermal delivery system rather than divided, as in an oral delivery system.

The compounds of this invention exhibit antiarrythmic activity useful in the treatment of various cardiac arrhythmias. The test procedures employed to measure this activity of the compounds of the present invention are described below.

Example 1

Guinea pigs, of either sex weighing between 200-350 g, are acutely sacrificed and the right ventricular papillary muscle is isolated. A sample of a given test compound is added using an in vitro tissue bath. Concentrations used are generally 3 x $10^{-5}$M, but may also be as low as 3 x $10^{-7}$M. Changes in refractory period are measured before and after adding 1 concentration (usually 3 x $10^{-5}$M, as noted above) of a test compound to the bath. One hour is allowed for drug equilibration. A compound is considered active (Class III) if an increase in ventricular refractory period is 25msec or more (at 3 x $10^{-5}$M .

Results

| Compound | Concentration (M) | Change (msec) |
|---|---|---|
| $H_2O$ | -- | 8 |
| Disopyramide | 3 x $10^{-5}$ | 20 |
| Clofinium | 3 x $10^{-5}$ | 24 |
| Sotalol | 3 x $10^{-5}$ | 35 |
| Example 19 | 1 x $10^{-6}$ | 25 |
| Example 20 | 1 x $10^{-6}$ | 40 |
| Example 21 | 3 x $10^{-5}$ | 190 |
| Example 24 | 3 x $10^{-5}$ | 60 |
| Example 25 | 3 x $10^{-5}$ | 60 |
| Example 26 | 3 x $10^{-5}$ | 90 |

| Compound | Results Concentration (M) | Change (msec) |
|---|---|---|
| Example 29 | $3 \times 10^{-6}$ | 60 |
| Example 87 | $3 \times 10^{-6}$ | 55 |
| Example 30 | $3 \times 10^{-6}$ | 80 |
| Example 36 | $3 \times 10^{-6}$ | 55 |
| Example 37 | $3 \times 10^{-5}$ | 35 |
| Example 39 | $3 \times 10^{-5}$ | 155 |
| Example 40 | $3 \times 10^{-5}$ | 125 |
| Example 41 | $3 \times 10^{-6}$ | 70 |
| Example 42 | $3 \times 10^{-6}$ | 60 |
| Example 44 | $3 \times 10^{-6}$ | 40 |
| Example 46 | $3 \times 10^{-6}$ | 95 |
| Example 51 | $3 \times 10^{-6}$ | 75 |
| Example 53 | $3 \times 10^{-6}$ | 50 |
| Example 58 | $3 \times 10^{-6}$ | 60 |
| Example 60 | $3 \times 10^{-6}$ | 25 |
| Example 59 | $3 \times 10^{-6}$ | 35 |
| Example 64 | $3 \times 10^{-5}$ | 85 |
| Example 66 | $3 \times 10^{-5}$ | 45 |
| Example 69 | $3 \times 10^{-5}$ | 25 |
| Example 70 | $3 \times 10^{-5}$ | 40 |
| Example 72 | $3 \times 10^{-5}$ | 50 |
| Example 75 | $3 \times 10^{-5}$ | 60 |
| Example 77 | $1 \times 10^{-6}$ | 60 |
| Example 78 | $3 \times 10^{-6}$ | 50 |
| Example 80 | $3 \times 10^{-5}$ | 115 |
| Example 82 | $3 \times 10^{-6}$ | 55 |
| Example 4 | $3 \times 10^{-5}$ | 35 |
| Example 7 | $3 \times 10^{-6}$ | 30 |
| Example 92 | $1 \times 10^{-5}$ | 45 |
| Example 93 | $3 \times 10^{-5}$ | 55 |
| Example 94 | $3 \times 10^{-6}$ | 40 |
| Example 96 | $3 \times 10^{-6}$ | 55 |
| Example 97 | $3 \times 10^{-5}$ | 55 |
| Example 99 | $3 \times 10^{-6}$ | 105 |
| Example 90 | $3 \times 10^{-6}$ | 50 |
| Example 91 | $3 \times 10^{-6}$ | 65 |
| Example 109 | $3 \times 10^{-5}$ | 55 |

The following non-limiting examples further illustrate details for the preparation of the compounds of the present invention. Those skilled in the art will readily understand that known variations of the conditions and processes of the following preparative procedures can be used to prepare these compounds. All termperatures are degrees Celsius unless otherwise noted. Melting points were determined on a Thomas-Hoover Unimelt Capillary Apparatus and are not corrected. Unless otherwise noted, I.R. and NMR spectra were consistent with the assigned structure.

EXAMPLE 2

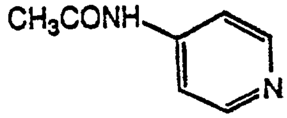

Preparation of 4-acetamidopyridine acetate II

4-Aminopyridine (101.28 g) and acetic anhyride (110 g) were mixed neat and heated at 100° C for 1/2 h. The solidified reaction mixture was triturated with acetone, filtered off, and washed with ether to afford 186.48 g of II as a white solid in two crops. Anal. calcd for $C_9H_{12}N_2O_3$: C, 55.09; H, 6.16; N, 14.26. Found: C, 55.04; H, 5.96; N, 15.22.

EXAMPLE 3

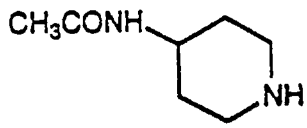

Preparation of 4-acetamidopiperidine acetate III

A solution of the product of Example 2 (75 g) in 750 mL acetic acid was reduced over $PtO_2$ catalyst at 60 psi hydrogen atmosphere at 60° C for 7 hours. The solution was filtered, concentrated and triturated with ether to afford the title compound quantitatively as a white solid which was used directly in subsequent reactions.

EXAMPLE 4

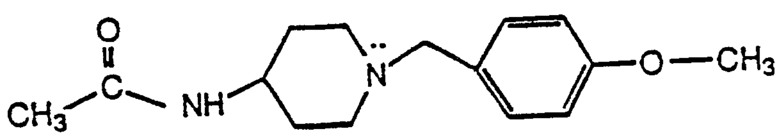

Preparation of 1-(4-methoxyphenyl)methyl-4-acetamido-piperidine

A mixture of 10 g amine acetate from Example 3 and 13.48 g 4-methoxy benzaldehyde was hydrogenated in 100 mL ethanol over a Pt/C catalyst at room temperature for 3 hours. The reaction mixture ws filtered and concentrated to give 74.0 g of the acetate salt of the title compound as a white solid which was hydrolyzed directly as described in Example 4. (An alternative reductive amination procedure is described in Example 5). Conversion of a sample to the free base using aqueous base and ethyl acetate extraction provided a white solid after solvent evaporation and trituration with ether: mp 140-142° C; Anal. calcd for $C_{15}H_{22}N_2O_2$: C, 68.67; H, 8.45; N, 10.68. Found: C, 65.26; H, 8.60; N, 10.77.

EXAMPLE 5

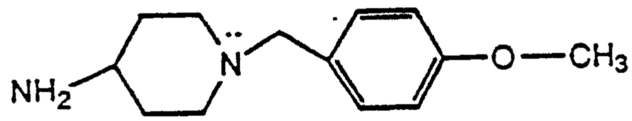

Preparation of 1-(4-methoxyphenyl)methyl-4-amino piperidine

A) A solution of 50 g of the product of Example 4 was dissolved in 500 mL of 1.2 N HCl and heated at 100 °C for 8 h. The solution was made alkaline with 50% aq. NaOH and extracted three times with ether. The combined organic layers were washed with water and saturated brine, dried over sodium sulfate, and concentrated to give the title compound as 28 g of clear oil which was used without further purification.

B) (Alternative general reductive alkylation procedure) A solution of 50 mmol amine acetate (product of Example 3) and 100 mmol of 4-methoxybenzaldehyde in 125 mL methylene chloride and 15 mL acetic acid was treated with 50 mmol of borane-pyridine complex and allowed to stir at room temperature overnight. The removal of volatiles by rotary evaporation afforded the acetamide of Example 4 as an oil which was dissolved in 300 mL of 1.2 N HCl and heated overnight on a steam bath. The cooled reaction mixture was extracted once with a 50 mL portion of ethyl acetate which was discarded. The aqueous layer was made basic with aq. NaOH and extracted three times with 50 mL ether. The combined layers were washed with water and dried over sodium sulfate. Solvent removal afforded the title compound as a crude oil (yield typically 60-70% for two steps) which was used directly without further purification.

## EXAMPLE 6

General acylation procedures

A) 10 mmol of the amine of Example 5 is dissolved in a mixture of 25 mL chloroform and 11 mmol of triethylamine cooled to 0 °C. A solution of 11 mmol of the acyl chloride neat or dissolved in 25 mL chloroform is added dropwise and the reaction mixture is allowed to stir for 1 h . Volatiles are removed in vacuo and the residue is partitioned between dilute aqueous base and ethyl acetate. Drying of the ethyl acetate extract and evaporation leads to the crude product which is optionally purified by flash chromatography on silica gel using 92.5: 7: 0.5 chloroform: ethanol: ammonium hydroxide and crystallized from ethyl acetate/hexane or converted to the HCl salt using dioxane/HCl followed by recrystallization from methanol/ether.

B) A stirred solution of 10 mmol acylating acid in 25 mL chloroform is treated with 10 mmol of triethylamine followed by 10 mmol of isobutyl chloroformate. After 10 minutes at ambient temperature the amine of Example 5 was added and the reaction is allowed to stir for 1/2 h. The reaction mixture is washed with 10% NaOH solution and the organic layer is dried and evaporated to give a residue which is optionally purified by flash chromatography on silica gel using 92.5:7:0.5 chloroform:ethanol:ammonium hydroxide recrystallized from ethyl acetate converted to the HCl salt using dioxane/HCl followed by recrystallization from methanol/ether.

## Example 7

Preparation of quaternary salt

A solution of 200 mg of the amide of Example 6 in 5 mL acetone was treated with 4 drops of iodomethane. The reaction mixture was stirred for 18 h and the white crystalline precipitate was filtered off to afford 206 mg of white solid which was recrystallized from acetonitrile to give 128 mg of quaternary iodide as fluffy white needles, mp 236-237 °C.

## Example 8

### Preparation of N-oxide

A solution of 0.50 g of the amide of Example 6 in 10 mL $CH_2Cl_2$ was treated with 300 mg of m-chloroperoxybenzoic acid at 0 °C. After 1 h the solution was washed consecutively with 10mL 1N NaOH, water, and sat'd. brine. The solution was dried over sodium sulfate and concentrated to afford 0.51 g of white solid which was recrystallized from $CH_2Cl_2$/ethyl acetate to give 0.33 g of an N-oxide as a white powder, mp 200.5-202.5 °C.

## Examples 9 through 91

Using the procedures of Examples 2 through 8 and making the appropriate substitutions at positions $R^1$, $R^2$, $R^3$, and

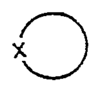 ,

the following products were obtained as presented below. The formula which precedes Example 9 specifies the moiety at $R^1$, $R^2$, $R^3$ and

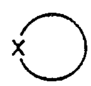 ,

the number of methylenes represented by n, the compound's melting point range in degrees Celsius (where available) and the compound's elemental analysis.

Again using the procedures of Examples 2 through 8 and making the appropriate substitutions at positions $R^1$, $R^2$, $R^3$ and $R^4$ of the general formula which is presented at the beginning of Example 92, the products of Examples 92 through 108 were obtained.

I

All piperidinyls are 4-piperidinyl unless otherwise noted.

For Examples 9 to 91 the Following Structure Applys

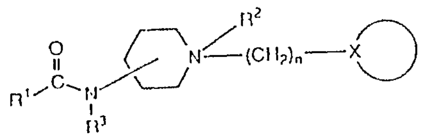

| Example | $R^1$ | $R^2$ | $R^3$ | X | n | mp, deg. C | Analysis |
|---|---|---|---|---|---|---|---|
| 9 | 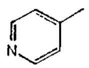 | | H | 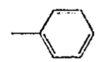 | 1 | | $C_{18}H_{21}N_3O$ |
| 10 | 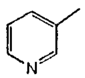 | | H | 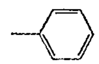 | 1 | | $C_{18}H_{21}N_3O$ |
| 11 | 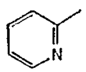 | | H | 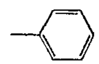 | 1 | | $C_{18}H_{21}N_3O$ |
| 12 | 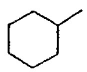 | | H | 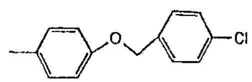 | 1 | | $C_{26}H_{33}ClN_2O_2$ |
| 13 | 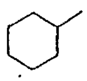 | | H | 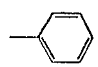 | 1 | 155-157 | $C_{19}H_{28}N_2O$ |
| 14 | 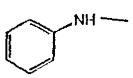 | | H | 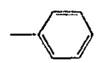 | 1 | 168-169.5 | $C_{19}H_{23}N_3O$ |
| 15 | 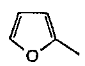 | | H | 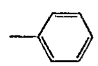 | 1 | 155.5-158 | $C_{17}H_{20}N_2O_2$ |

| Example | $R^1$ | $R^2$ | $R^3$ | X | n | mp, deg. C | Analysis |
|---|---|---|---|---|---|---|---|
| 16 | 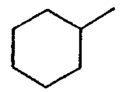 | | H | 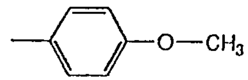 | 1 | 170-171 | $C_{20}H_{30}N_2O_2$ |
| 17 | $-CH_3$ | | H | 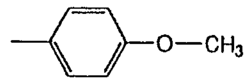 | 1 | 137-140 | $C_{15}H_{22}N_2O_2$ |
| 18 | 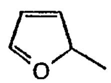 | | H | 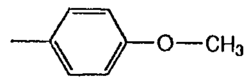 | 1 | 136-137 | $C_{18}H_{22}N_2O_3$ |
| 19 | 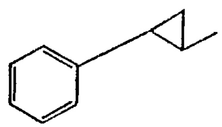 | | H | 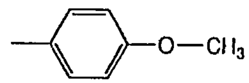 | 1 | 142-143 | $C_{23}H_{28}N_2O_2$ |
| 20 | 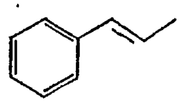 | | H | 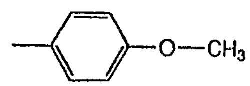 | 1 | 135-137 | $C_{22}H_{26}N_2O_2$ |
| 21 | 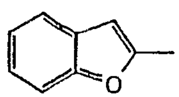 | | H | 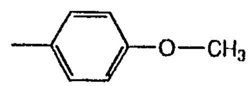 | 1 | 136-138 | $C_{22}H_{24}N_2O_3$ |

| Example | $R^1$ | $R^2$ | $R^3$ | X | n | mp, deg. C | Analysis |
| --- | --- | --- | --- | --- | --- | --- | --- |
| 22 | 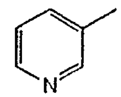 | | H | 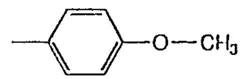 | 1 | | $C_{19}H_{23}N_3O_2$ |
| 23 | 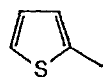 | | H | 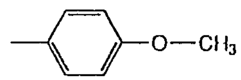 | 1 | 161-162 | $C_{18}H_{22}N_2O_2S$ |
| 24 | 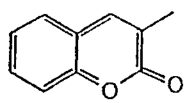 | | H | 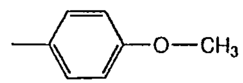 | 1 | 200-202 | $C_{23}H_{24}N_2O_4$ |
| 25 | 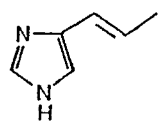 | | H | 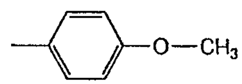 | 1 | 210-211 | $C_{19}H_{24}N_2O_2$ |
| 26 | 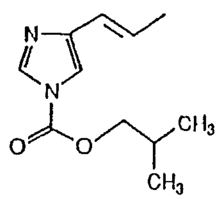 | | H | 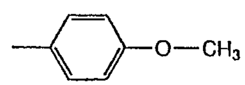 | 1 | 134-137 | $C_{24}H_{32}N_2O_4$ |
| 27 | $CH_3$ | | H | 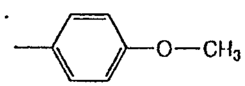 | 2 | 162-164 | $C_{16}H_{24}N_2O_2$ |

| Example | $R^1$ | $R^2$ | $R^3$ | X | n | mp, deg. C | Analysis |
|---|---|---|---|---|---|---|---|
| 28 | 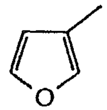 | | H | 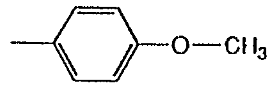 | 1 | 178.5-179.5 | $C_{18}H_{22}N_2O_2$ |
| 29 | 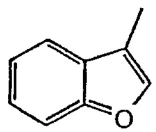 | | H | 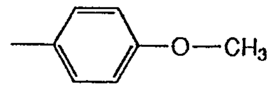 | 1 | 140-141 | $C_{22}H_{24}N_2O_3$ |
| 30 | 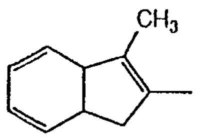 | | H | 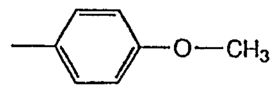 | 1 | 135-137 | $C_{23}H_{28}N_2O_2$ |
| 31 | 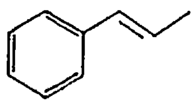 | | H | 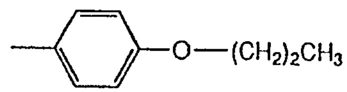 | 1 | 156-158 | $C_{24}H_{30}N_2O_2$ |
| 32 | 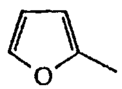 | | H | 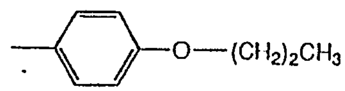 | 1 | 122-124 | $C_{20}H_{26}N_2O_3$ |
| 33 | 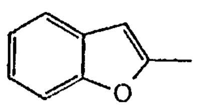 | | H | 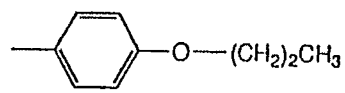 | 1 | 132-134 | $C_{24}H_{28}N_2O_3$ |

| Example | $R^1$ | $R^2$ | $R^3$ | X | n | mp, deg. C | Analysis |
|---|---|---|---|---|---|---|---|
| 34 | 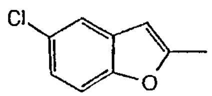 | | H | 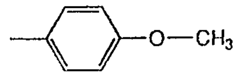 | 1 | 148-149 | $C_{22}H_{23}ClN_2O_3$ |
| 35 | 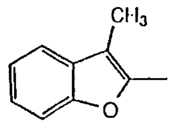 | | H | 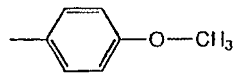 | 1 | 156-158 | $C_{23}H_{26}N_2O_2$ |
| 36 | 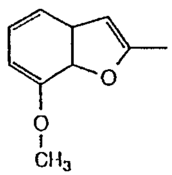 | | H | 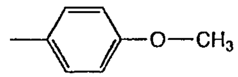 | 1 | 140-142 | $C_{23}H_{26}N_2O_4 \cdot HCl$ |
| 37 | 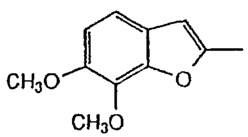 | | H | 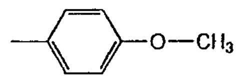 | 1 | 147-148 | $C_{24}H_{28}N_2O_5$ |
| 38 | 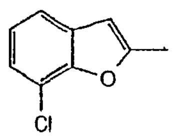 | | H | 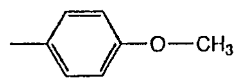 | 1 | 126-128 | $C_{22}H_{23}ClN_2O_3$ |
| 39 | 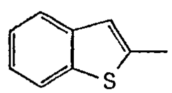 | | H | 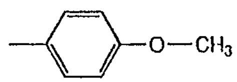 | 1 | 158-160 | $C_{22}H_{24}N_2O_2S$ |

| Example | $R^1$ | $R^2$ | $R^3$ | X | n | mp, deg. C | Analysis |
|---|---|---|---|---|---|---|---|
| 40 | 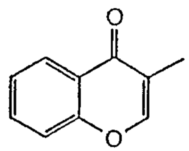 | | H | 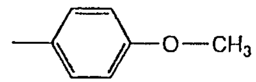 | 1 | 270-272 | $C_{23}H_{24}N_2O_4 \cdot HCl$ |
| 41 | 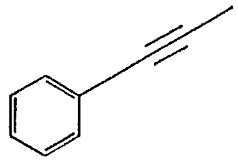 | | H | 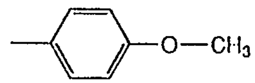 | 1 | oil | $C_{22}H_{25}N_2O_2 \cdot HCl$ |
| 42 | 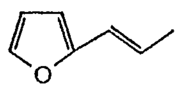 | | H | 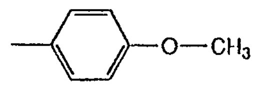 | 1 | 146-148 | $C_{23}H_{28}N_2O_3$ |
| 43 | 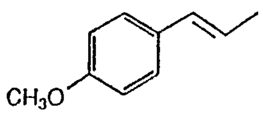 | | H | 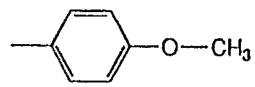 | 1 | 147-149 | $C_{23}H_{28}N_2O_3$ |
| 44 | 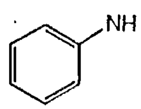 | | H | 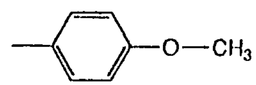 | 1 | 145-147 | $C_{20}H_{25}N_3O_2$ |
| 45 | 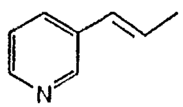 | | H | 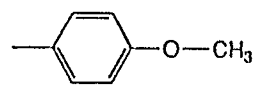 | 1 | 150-152 | $C_{21}H_{25}N_3O_2$ |

| Example | $R^1$ | $R^2$ | $R^3$ | X | n | mp, deg. C | Analysis |
|---|---|---|---|---|---|---|---|
| 46 | 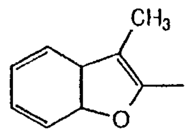 | | H | 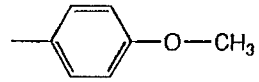 | 1 | 142-144 | $C_{23}H_{26}N_2O_3$ |
| 47 | 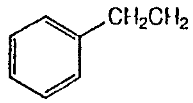 | | H | 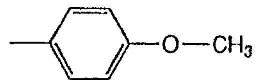 | 1 | 131-133 | $C_{22}H_{28}N_2O_2$ |
| 48 | 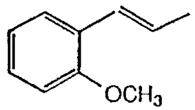 | | H | 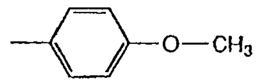 | 1 | 141-143 | $C_{23}H_{28}N_2O_2$ |
| 49 | 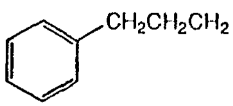 | | H | 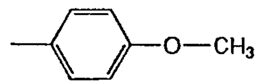 | 1 | 125-127 | $C_{23}H_{30}N_2O_2 \cdot HCl$ |
| 50 | 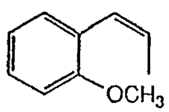 | | H | 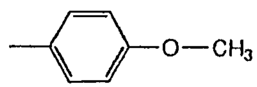 | 1 | 92-96 | $C_{23}H_{28}N_2O_2 \cdot HCl'$ |
| 51 | (±) 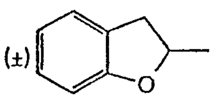 | | H | 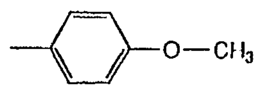 | 1 | 134.5-135 | $C_{22}H_{26}N_2O_3$ |

| Example | $R^1$ | $R^2$ | $R^3$ | X | n | mp, deg. C | Analysis |
|---|---|---|---|---|---|---|---|
| 52 | 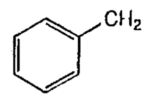 | | H | 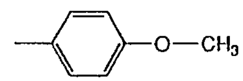 | 1 | | $C_{21}H_{26}N_2O_2$ |
| 53 | 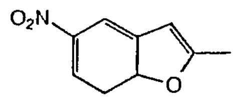 | | H | 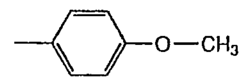 | 1 | 190-192 | $C_{22}H_{23}N_3O_5$ |
| 54 | 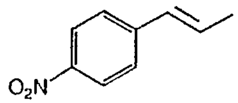 | | H | 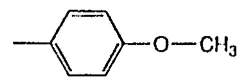 | 1 | 197-199 | $C_{22}H_{25}N_3O_4$ |
| 55 | 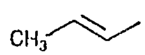 | | H | 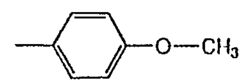 | 1 | | $C_{17}H_{24}N_2O_2$ |
| 56 | 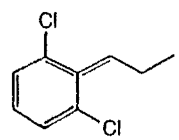 | | H | 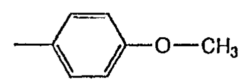 | 1 | 210-212 | $C_{22}H_{24}Cl_2N_2O_2$ |
| 57 | 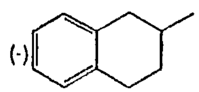 | | H | 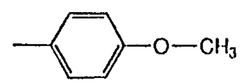 | 1 | 142-143 | $C_{24}H_{30}N_2O_2$ |
| 58 | 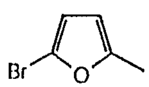 | | H | 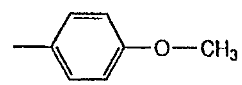 | 1 | 133-135 | $C_{18}H_{21}BrN_2O_3$ |

| Example | $R^1$ | $R^2$ | $R^3$ | X | n | mp, deg. C | Analysis |
|---|---|---|---|---|---|---|---|
| 59 | 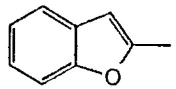 | | H | 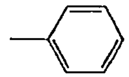 | 1 | 155-157 | $C_{21}H_{22}N_2O_3$ |
| 60 | 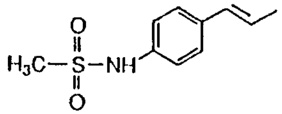 | | H | 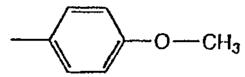 | 1 | 215-218 | $C_{23}H_{29}N_3O_4S$ |
| 61 | $CH_3$ | | H | 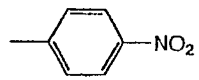 | 1 | 143-144 | $C_{14}H_{19}N_3O_3$ |
| 62 | 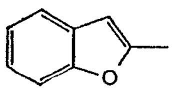 | | H | 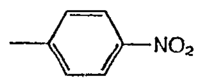 | 1 | 155-157 | $C_{21}H_{21}N_3O_4$ |
| 63 | $CH_3$ | | H | 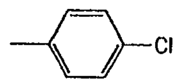 | 1 | 162-164 | $C_{14}H_{19}ClN_2O$ |
| 64 | 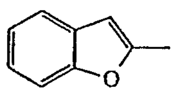 | | H | 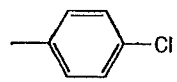 | 1 | 137-139 | $C_{21}H_{21}ClN_2O_2$ |
| 65 | (+) 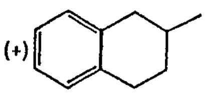 | | H | 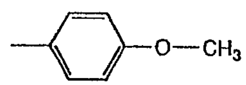 | 1 | 157-159 | $C_{24}H_{30}N_2O_2$ |

| Example | $R^1$ | $R^2$ | $R^3$ | X | n | mp, deg. C | Analysis |
|---|---|---|---|---|---|---|---|
| 66 | 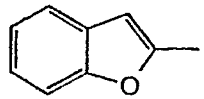 | $CH_3$ | H | 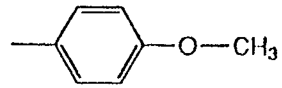 | 1 | 236-237 | $C_{23}H_{26}N_2O_3 \cdot HI$ |
| 67 | 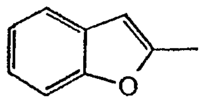 | | H | 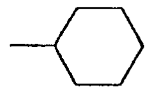 | 1 | 184-185.5 | $C_{21}H_{28}N_2O_2$ |
| 68 | $CH_3$ | | H | 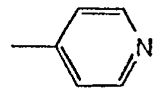 | 1 | 163-164.5 | $C_{13}H_{19}N_3O$ |
| 69 | $CH_3$ | | H | (3-piperidyl) 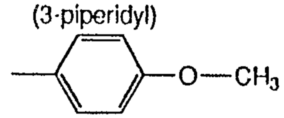 | 1 | 103-105 | $C_{15}H_{22}N_2O_2$ |
| 70 | 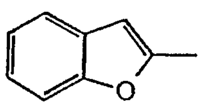 | | H | (3-piperidyl) 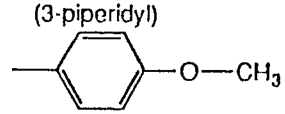 | 1 | 114-115 | $C_{22}H_{24}N_2O_3$ |
| 71 | $CH_3$ | | H | 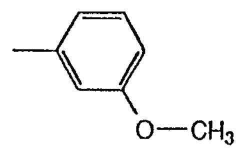 | 1 | | $C_{15}H_{22}N_2O_2$ |
| 72 | 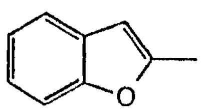 | O | H | 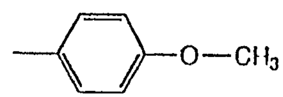 | 1 | 200.5-202.5 | $C_{22}H_{24}N_2O_4$ |

| Example | $R^1$ | $R^2$ | $R^3$ | X | n | mp, deg. C | Analysis |
|---|---|---|---|---|---|---|---|
| 73 | 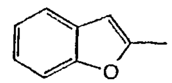 | | H | H | 1 | | $C_{15}H_{18}N_2O_2$ |
| 74 | $CH_3$ | | H | 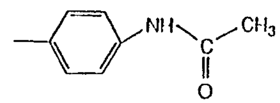 | 1 | 145-147 | $C_{16}H_{23}N_3O_2$ $\cdot CHOOOH$ |
| 75 | 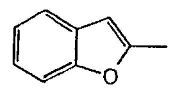 | | H | 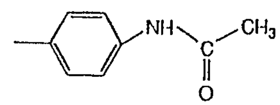 | 1 | 230-232 | $C_{23}H_{25}N_3O_3$ |
| 76 | 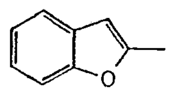 | | H | 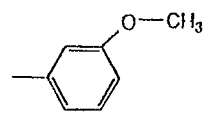 | 1 | | $C_{22}H_{25}ClN_2O_3$ |
| 77 | 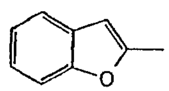 | | H | 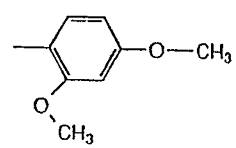 | 1 | | $C_{23}H_{26}N_2O_4$ |
| | 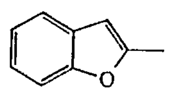 | | H | 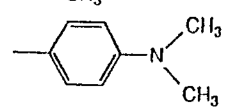 | 1 | | $C_{23}H_{27}N_3O_2\cdot 2(HCl)$ |
| 79 | 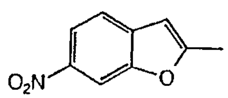 | | H | 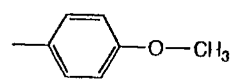 | 1 | 189-190 | $C_{22}H_{23}N_3O_5$ |
| 80 | 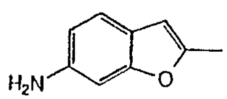 | | H | 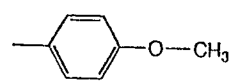 | 1 | 98-100 | $C_{22}H_{27}N_3O_3\cdot 2(HCl)$ |

| Example | $R^1$ | $R^2$ | $R^3$ | X | n | mp, deg. C | Analysis |
|---|---|---|---|---|---|---|---|
| 81 | $CH_3$ | | H | 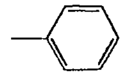 | 1 (substituted by $CO_2CH_2CH_3$) | | $C_{17}H_{24}N_2O_3$ |
| 82 | 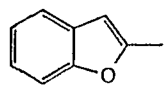 | | $-CH_2CO_2CH_2CH_3$ | 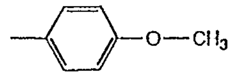 | 1 | | $C_{26}H_{31}ClN_2O_5$ |
| 83 | 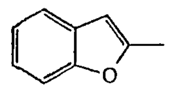 | | $-CH_2CO_2H$ | 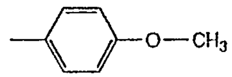 | 1 | | $C_{24}H_{26}N_2O_5$ |
| 84 | (±) 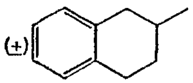 | | H | 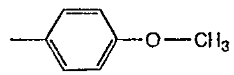 | 1 | 152-153 | $C_{24}H_{30}N_2O_2$ |
| 85 | 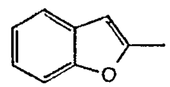 | | H | 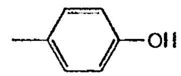 | 1 | 198-200 | $C_{21}H_{22}N_2O_3$ |
| 86 | $CH_3$ | | H | 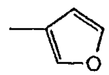 | 1 | | $C_{12}H_{18}N_2O_2$ |
| 87 | 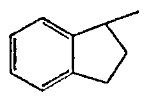 | | H | 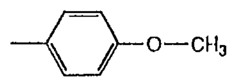 | 1 | | $C_{23}H_{28}N_2O_2$ |
| 88 | 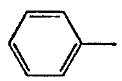 | | H | 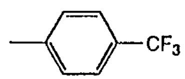 | 1 | | $C_{20}H_{22}N_2OF_3$ |
| 89 | 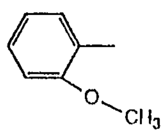 | | H | 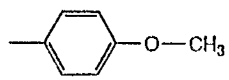 | 1 | | $C_{21}H_{27}N_2O_3$ |

| Examplo | $R^1$ | $R^2$ | $R^3$ | X | n | mp, deg. C | Analysis |
|---|---|---|---|---|---|---|---|
| 90 | 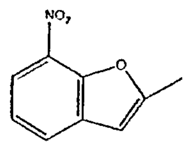 | H | H | 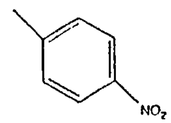 | 1 | | $C_{21}H_{20}N_4O_6$ |
| 91 | 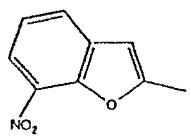 | H | H | 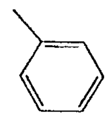 | 1 | | $C_{21}H_{21}N_3O_4$ |

For Examples 92 to 108 the Following Structure Applys

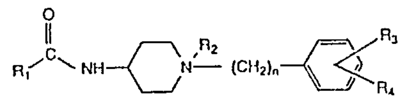

| Example | $R^1$ | $R^2$ | $R^3, R^4$ | n | mp. deg. C | Analysis |
|---|---|---|---|---|---|---|
| 92 | 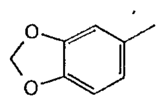 | | 4-$OCH_3$ | 1 | 154-156 | $C_{21}H_{24}N_2O_4$ |
| 93 | 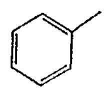 | | 3,4($OCH_3$)$_2$ | 1 | 154-155 | $C_{21}H_{26}N_2O_3$ |
| 94 | 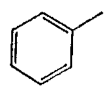 | | 4-$OCH_3$ | 2 | 171-172 | $C_{21}H_{26}N_2O_2$ |
| 95 | 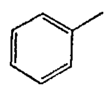 | | 4-$OCH_2CH_2CH_3$ | 1 | 155-157 | $C_{22}H_{28}N_2O_2$ |
| 96 | 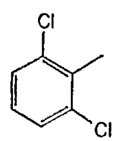 | | 4-$OCH_3$ | 1 | 135-138 | $C_{20}H_{22}Cl_2N_2O_2$ |
| 97 | 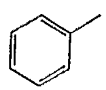 | $CH_3$ | 4-$OCH_3$ | 1 | 218-220 | $C_{21}H_{27}IN_2O_2$ |

| Example | $R^1$ | $R^2$ | $R^3$, $R^4$ | n | mp, deg. C | Analysis |
|---|---|---|---|---|---|---|
| 98 | 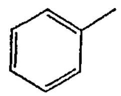 | | 4-$CF_3$ | 1 | 203.5-204.5 | $C_{20}H_{21}F_3N_2O$ |
| 99 | 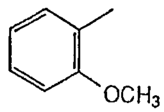 | | 4-$OCH_3$ | 1 | oil | $C_{21}H_{26}N_2O_3$ |
| 100 | 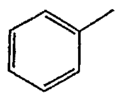 | | 4-$OCH_2$— 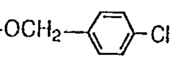 | 1 | | $C_{26}H_{27}ClN_2O_2$ |
| 101 | 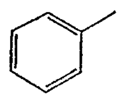 | | | 1 | 166-168 | $C_{19}H_{22}N_2O$ |
| 102 | 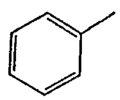 | | 4-$OCH_3$ | 1 | 157-159 | $C_{20}H_{24}N_2O_2$ |

| Example | $R^1$ | $R^2$ | $R^3$, $R^4$ | n | mp, deg. C | Analysis |
|---|---|---|---|---|---|---|
| 103 | 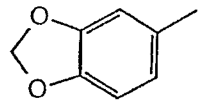 | | | 1 | 162-164 | $C_{20}H_{22}N_2O_3$ |
| 104 | 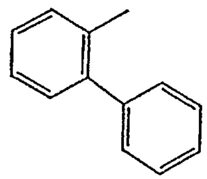 | | | 1 | 143.5-145.0 | $C_{25}H_{26}N_2O$ |
| 105 | 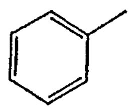 | | 4-Cl | 1 | 180-182 | $C_{19}H_{21}N_2O$ |
| 106 | 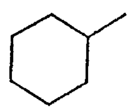 | | | 1 | 155-157 | $C_{19}H_{28}N_2O$ |
| 107 | 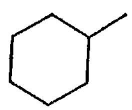 | | 4-$OCH_3$ | 1 | 170-171 | $C_{20}H_{30}N_2O_2$ |

| Example | $R^1$ | $R^2$ | $R^3$, $R^4$ | n | mp, deg. C | Analysis |
|---|---|---|---|---|---|---|
| 108 | 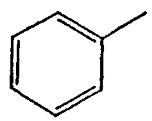 | | 4-$OCH_2$— 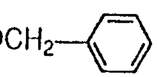 | | 148-151 | $C_{26}H_{28}N_2O_2$ |

## Example 109

Preparation of

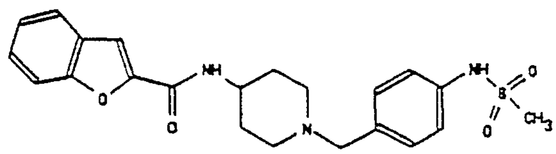

N-[1-[[4-[(methylsulfonyl)amino]phenyl]methyl]-4-piperidinyl]-2-benzofurancarboxamide

To a solution of Example 62 (1.2g, 3.16 mmol) in ethanol (10 ml) and concentrated hydrochloric acid (10 ml), tin (11) chloride monohydrate (2.15g, 9.5 mmol) was added. The resulting solution was heated (steam bath) for 30 min., cooled to room temperature and adjusted to pH 10.0 by careful addition of 1N potassium hydroxide solution. The resulting aqueous solution was extracted with methylene chloride. The organic extracts were separated; dried ($Na_2SO_4$) and evaporated to afford the crude amino derivative (820 mg).

To a stirred solution of the amino derivative (349 mg) in methylene chloride (10 ml) at -78°, triethylamine (0.5 ml) and methane sulphonyl chloride (0.1 ml) was added. The reaction mixture was allowed to attain room temperature and stirred for a further 15 mins. The reaction mixture was quenched with saturated $NaHCO_3$ solution and extracted with methylene chloride. The organic extracts were separated, dried ($Na_2SO_4$) and evaporated in vacuo to afford the claimed compound (340 mg) recrystallized from ethylacetate, methylene chloride, methanol).

Anal. calcd for $C_{22}H_{25}N_3O_4S$, 0.6 $CH_2CL_2$; C 56.73, H, 5.52, N, 8.78.
Found C, 56.39, H, 5.45, N, 80.71.

## Example 110

Preparation of

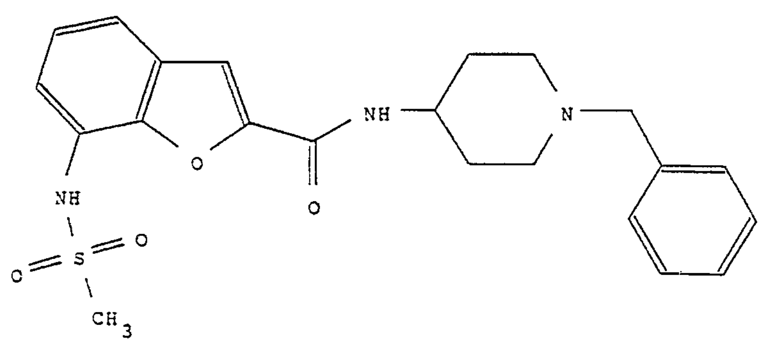

7-[(methylsulfonyl)amino]-N-[1-(phenylmethyl)-4-piperidinyl]-2-benzofurancarboxamide

Following the procedure of Example 109 the final product of Example 91 was converted to the title

compound.
Anal. calcd for $C_{22}H_{25}N_3O_4S$, C, 61.81, H, 5.89, N, 9.83.
Found, C, 62.08, H, 5.89, N, 9.91.

## Example 111

Preparation of

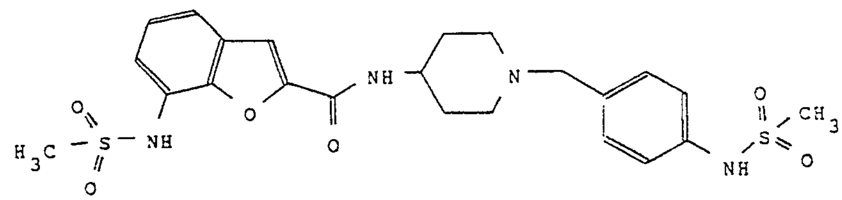

7-[(methylsulfonyl)amino]-N-[1-[[4-[(methylsulfonyl)amino]phenyl]methyl]-4-piperidinyl]-2-benzofurancarboxamide

Following the procedure of Example 109 the final product of Example 90 was converted to the title compound.
Cald. for $C_{22}H_{25}N_3O_4S$, $1H_2O$, 1/4 EtOAc, C, 51.42, H, 5.75, N, 9.99, S, 11.44.
Found C, 51.57, H, 5.50, N, 9.63, S, 11.52.

While the invention has been described and illustrated with reference to certain preparative embodiments thereof, those skilled in the art will appreciate that various changes, modifications and substitutions can be made therein without departing from the spirit and scope of the invention. For example, effective dosages other than the preferred range as set forth herein above may be applicable as a consequence of variations in the responsiveness of the mammal being treated for severity of cardiac arrhythmia, dosage-related adverse effects, if any, and analogous considerations. Likewise, the specific pharmacological responses observed may vary according to and depending upon the particular active compounds selected or whether there are present certain pharmaceutical carriers, as well as the type of formulation and mode of administration employed, and such expected variations for differences in the results are contemplated in accordance with the objects and practices of the present invention. It is intended, therefore, that the invention be limited only by the scope of the claims which follow, and that such claims be interpreted as broadly as is reasonable.

## Claims

1. A compound of the general formula

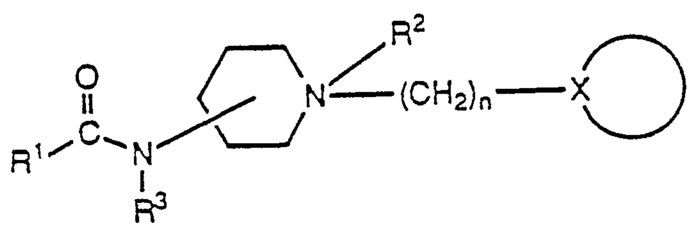

I

and the pharmaceutically acceptable non-toxic salts thereof, and the hydrated forms thereof, wherein $R^1$ is

alkenyl or alkynyl of from one to ten carbon atoms; substituted or unsubstituted aralkenyl or aralkynyl of from one to ten carbon atoms and wherein said aryl substituent is one or more of alkoxy, nitro, halogen or alkylsulfonamide at any position with alkoxy or alkylsulfonamide of one to ten carbon atoms; arylamino; aryl fused to substituted or unsubstituted cycloalkyl of from three to eight carbon atoms wherein said cycloalklyl substituent can be halogen; arylcycloalkyl wherein cycloalkyl is from three to eight carbon atoms; substituted or unsubstituted benzofuranyl wherein said benzofuranyl substituent can be one or more of halogen, amino, nitro, alkoxy of from one to ten carbon atoms or alkylsulfonamido wherein the alkyl portionis 1 to 4 carbon atoms at any position; substituted or unsubstituted benzopyran wherein said substituent can be keto; thiophene; benzothiophene; substituted or unsubstituted indene or isoindene wherein said substituent is alkyl of one to ten carbon atoms; or substituted or unsubstituted unsaturated cycloheteroalkenyl wherein the cyclohetero ring consists of 4 to 6 carbon atoms wherein one or two of the said carbon atoms are replaced by nitrogen or oxygen and the substituents are selected from the group consisting of alkyl of from one to ten carbon atoms, halogen, amino, alkoxy of from one to ten carbon atoms or alkylcarboxylate wherein the alkyl is 1 to 6 carbon atoms; substituted or unsubstituted unsaturated cycloheteroalkynyl wherein the cyclohetero ring consists of 4 to 6 carbon atoms wherein one or two of the said carbon atoms are replaced by nitrogen or oxygen and the substituents are the same as those already described for the unsaturated cycloheteroalkenyl; with the proviso that when $R^2$ is alkyl of 1 to 10 carbon atoms or oxygen $R^1$ can also be aryl or substituted aryl wherein said aryl substituent is one or more of alkyl of from one to ten carbon atoms, halogen, amino or alkoxy of from one to ten carbon atoms.

n is an integer of from one to ten;

$R^2$ is unsubstituted or is alkyl of from one to ten carbon atoms or oxygen that is present as an N-oxide;

$R^3$ is hydrogen, carboxyalkyl of from one to ten carbon atoms or alkoxycarbonylalkyl of from one to ten carbon atoms;

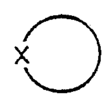

is hydrogen; pyridinyl; cycloalkyl of three to eight carbon atoms or hydroxy substituted cycloalkyl of three to eight carbon atoms; furanyl; or unsubstituted or substituted phenyl wherein said phenyl substituent is one or more of alkyl, halogen substituted alkyl of one to ten carbon atoms, alkoxy from one to ten carbon atoms, nitro, amine, mono or dialkylamine, acetyl amine, acetylamide, halogen, alkylsulfonamido wherein the alkyl portion is 1 to 4 carbon atoms, alkoxy itself substituted by halogen substituted phenyl or aryalkoxy of from one to ten carbon atoms in length wherein aryl may be substituted by halogen or alkyl of one to ten carbon atoms in length.

2. A compound according to Claim 1 which is selected from the group consisting of
N-[1-[(4-methoxyphenyl)methyl]-4-piperidinyl]2E-buteneamide
N-[1-[(4-methoxyphenyl)methyl]-4-piperidinyl]-3-phenyl-2E-propenamide
3-phenyl-N-[1-[(propoxyphenyl)methyl]-4-piperidinyl]-2E-propenamide
3-(2-methoxyphenyl)-N-[1-[(4-methoxyphenyl)methyl]-4-piperidinyl]-2Z-propenamide
3-(2-methoxyphenyl)-N-[1-[(4-methoxyphenyl)methyl]-4-piperidinyl-2Z-propenamide, monohydrochloride 3-(4-methoxyphenyl)-N-[1-[(4-methoxyphenyl)methyl]-4-piperidinyl]-2E-propenamide
N-[1-[(4-methoxyphenyl)methyl]-4-piperidinyl]-3-(4-nitrophenyl)-2E-propenamide
N-[1-[(4-methoxyphenyl)methyl]-4-piperidinyl]-3-[4-[(methylsulfonyl)amino]phenyl]-2E-propenamide
3-(2,6-dichlorophenyl)-N-[1-[(4-methoxyphenyl)methyl] -4-piperidinyl]-2E-propenamide
3-(2-furanyl)-N-[1-[(4-methoxyphenyl)methyl]-4-piperidinyl]-2E-propenamide
3-(1H-imidazol-4-yl)-N-[(4-methoxyphenyl)methyl]-4-piperidinyl]-2E-propenamide
2-methylpropyl 4-[3-[[1-[(4-methoxyphenyl)methyl]-4-piperidinyl]amino]-3-oxo-1E-propenyl]-1H-imidazole-1-carboxylate
N-[1-[(4-methoxyphenyl)methyl]-4-piperidinyl]-3-(3-pyridinyl)-2E-propenamide
N-[1-[(4-methoxyphenyl)methyl]-4-piperidinyl]-3-phenyl-2-propynamide, monohydrochloride
N-[1-[(4-methoxyphenyl)methyl]-4-piperidinyl]-2-phenylcyclopropanecarboxamide
2,3-dihydro-N-[1-[(4-methoxyphenyl)methyl]-4-piperidinyl]-1H-indene-1-carboxamide
N-[1-[(4-methoxyphenyl)methyl]-4-piperidinyl]-3-methyl-1H-indene-2-carboxamide
1,2,3,4-tetrahydro-N-[1-[(4-methoxphenyl)methyl]-4-piperidinyl]-2-naphthalenecarboxamide
(-)1,2,3,4-tetrahydro-N-[1-[(4-methoxyphenyl)methyl]-4-piperidinyl]-2-naphthalenecarboxamide

(+)1,2,3,4-tetrahydro-N-[1-[(4-methoxyphenyl)methyl]-4-piperidinyl]-2-naphthalenecarboxamide
2,3-dihydro-N-[1-[(4-methoxyphenyl)methyl]-4-piperidinyl]-2-benzofurancarboxamide
N-(1-methyl-4-piperidinyl)-2-benzofurancarboxamide
N-[1- cyclohexylmethyl)-4-piperidinyl]-2-benzofurancarboxamide
N-[1-(phenylmethyl)-4-piperidinyl]-2-benzofurancarboxamide
N-[1-[(4-chlorophenyl)methyl]-4-piperidinyl)-2-benzofurancarboxamide
N-[1-[(4-hydroxyphenyl)methyl]-4-piperidinyl]-2-benzofurancarboxamide
N-[1-[(4-methoxyphenyl)methyl]-4-piperidinyl]-2-benzofurancarboxamide
N-[1-[(4-methoxyphenyl)methyl]-4-piperidinyl]-3-benzofurancarboxamide
N-[1-[(4-methoxyphenyl)methyl]-3-piperidinyl-2-benzofurancarboxamide
4-[(2-benzofuranylcarbonyl)amino]-1-[(4-methoxyphenyl) methyl]-1-methylpiperidinium iodide
N-[1-[(4-methoxyphenyl)methyl]-4-piperidinyl]-2-benzofurancarboxamide, N-oxide
N-[1-[(4-propoxyphenyl)methyl]-4-piperidinyl]-2-benzofurancarboxamide
N-[1-[(4-nitrophenyl)methyl]-4-piperidinyl]-2-benzofurancarboxamide
N-[1-[[4-(acetylamino)phenyl]methyl]-4-piperidinyl]-2-benzofurancarboxamide
2-methyl-N-[1-[(4-methoxyphenyl)methyl]-4-piperidinyl]-2-benzofurancarboxamide
7-chloro-N-[1-[(4-methoxyphenyl)methyl]-4-piperidinyl]-2-benzofurancarboxamide
5-chloro-N-[1-[(4-methoxyphenyl)methyl]-4-piperidinyl]-2-benzofurancarboxamide
6-amino-N-[1-[(4-methoxyphenyl)methyl]-4-piperidinyl] -2-benzofurancarboxamide
N-[1-[(4-methoxyphenyl)methyl]-4-piperidinyl]-5-nitro -2-benzofurancarboxamide
N-[1-[(4-methoxyphenyl)methyl]-4-piperidinyl]-6-nitro -Z-benzofurancarboxamide
N-[1-[(3-methoxyphenyl)methyl]-4-piperidinyl]-2-benzofurancarboxamide
N-[1-[(2,4-dimethoxphenyl)methyl]-4-piperidinyl]-2-benzofurancarboxamide
N-[1-[(4-N,N-dimethylaminophenyl)methyl]-4-piperidinyl]-2-benzofurancarboxamide
N-[l-[(4-methoxyphenyl)methyl]-4-piperidinyl]-6-nitro -2-benzofurancarboxamide
N-[1-[(3-methoxyphenyl)methyl]-4-piperidinyl]-2-benzofurancarboxamide
N-[1-[(2,4-dimethoxyphenyl)methyl]-4-piperidinyl]-2-benzofurancarboxamide
N-[1-[(4-N,N-dimethylaminophenyl)methyl]-4-piperidinyl]-2-benzofurancarboxamide
7-methoxy-N-[1-[(4-methoxyphenyl)methyl]-4-piperidinyl]-2-benzofurancarboxamide, monohydrochloride
6,7-dimethoxy-N-[1-[(4-methoxyphenyl)methyl]-4-piperidinyl]-2-benzofurancarboxamide
N-[1-[(4-methoxyphenyl)methyl]-4-piperidinyl]-2-oxo-2H-1-benzopyran-3-carboxamide
N-[1-[(4-methoxyphenyl)methyl]-4-piperidinyl]-4-oxo-4H-1-benzopyran-3-carboxamide, monohydrochloride
N-[1-[(4-methoxyphenyl)methyl]-4-piperidinyl]-2-thiophenecarboxamide
N-[1-[(4-methoxyphenyl)methyl]-4-piperidinyl]-2-benzo[b]thiophenecarboxamide
N-[1-[[4-[(4-chlorophenyl)methoxy]phenyl]methyl]-4-peperidinyl]benzamide
N-[1-(phenylmethyl)-4-piperidinyl]-1,3-benzodioxole-5-carboxamide
N-[1-[(4-methoxyphenyl)methyl]-4-piperidinyl]-1,3-benzodioxole-5-carboxamide
2,6-dichloro-N-[1-[(4-methoxyphenyl)methyl]-4-piperidinyl]benzamide
4-(benzoylamino)-1-[(4-methoxyphenyl)methyl]-1-methylpiperidinium, iodide
7-nitro-N-[1-(phenylmethyl)-4-piperidinyl]-2-benzofurancarboxamide
7-nitro-N-[1-[(4-nitrophenyl)methyl]-4-piperidinyl]-2-benzofurancarboxamide
N-[1-[[4-[(methylsulfonyl)amino]phenyl]methyl]-4-piperidinyl]-2-benzofurancarboxamide
7-[(methylsulfonyl)amino]-N-[1-(phenylmethyl)-4-piperidinyl]-2-benzofurancarboxamide
7-[(methylsulfonyl)amino]-N-[1-[[4-[(methylsulfonyl)amino]phenyl]methyl]-4-piperidinyl]-2-benzofurancarboxamide

3. A pharmaceutical composition comprised of a pharmaceutically acceptable carrier in combination with a compound according to Claim 1.

4. A composition according to Claim 3 wherein the compound is selected from the group consisting of
N-[1-[(4-methoxyphenyl)methyl]-4-piperidinyl] 2E-buteneamide
N-[1-[(4-methoxyphenyl)methyl]-4-piperidinyl]-3-phenyl-2E-propenamide
3-phenyl-N-[1-[(propoxyphenyl)methyl]-4-piperidinyl]-2E-propenamide
3-(2-methoxyphenyl)-N-[1-[(4-methoxyphenyl)methyl]-4-piperidinyl]-2Z-propenamide
3-(2-methoxyphenyl)-N-[1-[(4-methoxyphenyl)methyl]-4-piperidinyl-2Z-propenamide, monohydrochloride
3-(4-methoxyphenyl)-N-[1-[(4-methoxyphenyl)methyl]-4-piperidinyl]-2E-propenamide
N-[1-[(4-methoxyphenyl)methyl]-4-piperidinyl]-3-(4-nitrophenyl]-2E-propenamide
N-[1-[(4-methoxyphenyl)methyl]-4-piperidinyl]-3-[4-[(methylsulfonyl)amino]phenyl]-2E-propenamide
3-(2,6-dichlorophenyl)-N-[1-[(4-methoxyphenyl)methyl] -4-piperidinyl]-2E-propenamide

3-(2-furanyl)-N-[1-[(4-methoxyphenyl)methyl]-4-piperidinyl]-2E-propenamide
3-(1H-imidazol-4-yl)-N-[(4-methoxyphenyl)methyl]-4-piperidinyl]-2E-propenamide
2-methylpropyl 4-[3-[[1-[(4-methoxyphenyl)methyl]-4-piperidinyl]amino]-3-oxo-1E-propenyl]-1H-imidazole1-carboxylate
N-[1-[(4-methoxyphenyl)methyl]-4-piperidinyl]-3-(3-pyridinyl)-2E-propenamide
N-[1-[(4-methoxyphenyl)methyl]-4-piperidinyl]-3-phenyl-2-propynamide, monohydrochloride
N-[1-[(4-methoxyphenyl)methyl]-4-piperidinyl]-2-phenylcyclopropanecarboxamide
2,3-dihydro-N-[1-[(4-methoxyphenyl)methyl]-4-piperidinyl]- 1H-indene-1-carboxamide
N-[1-[(4-methoxyphenyl)methyl]-4-piperidinyl]-3-methyl-1H-indene-2-carboxamide
1,2,3,4-tetrahydro-N-[1-[(4-methoxphenyl)methyl]-4-piperidinyl]-2-naphthalenecarboxamide
(-)1,2,3,4-tetrahydro-N-[1-[(4-methoxyphenyl)methyl]-4-piperidinyl]-2-naphthalenecarboxamide
(+)l,2,3,4-tetrahydro-N-[1-[(4-methoxyphenyl)methyl]-4-piperidinyl]-2-naphthalenecarboxamide
2,3-dihydro-N-[1-[(4-methoxyphenyl)methyl]-4-piperidinyl]-2-benzofurancarboxamide
N-(1-methyl-4-piperidinyl)-2-benzofurancarboxamide
N-[1-(cyclohexylmethyl)-4-piperidinyl]-2-benzofurancarboxamide
N-[1-(phenylmethyl)-4-piperidinyl]-2-benzofurancarboxamide
N-[1-[(4-chlorophenyl)methyl]-4-piperidinyl)-2-benzofurancarboxamide
N-[1-[(4-hydroxyphenyl)methyl]-4-piperidinyl]-2-benzofurancarboxamide
N-[1-[(4-methoxyphenyl)methyl]-4-piperidinyl]-2-benzofurancarboxamide
N-[1-[(4-methoxyphenyl)methyl]-4-piperidinyl]-3-benzofurancarboxamide
N-[1-[(4-methoxyphenyl)methyl]-3-piperidinyl-2-benzofurancarboxamide
4-[(2-benzofuranylcarbonyl)amino]-1-[(4-methoxyphenyl)methyl]-1-methylpiperidinium iodide
N-[1-[(4-methoxyphenyl)methyl]-4-piperidinyl]-2-benzofurancarboxamide, N-oxide
N-[1-[(4-propoxyphenyl)methyl]-4-piperidinyl]-2-benzofurancarboxamide
N-[1-[(4-nitrophenyl)methyl]-4-piperidinyl]-2-benzofurancarboxamide
N-[1-[[4-(acetylamino)phenyl]methyl]-4-piperidinyl]-2-benzofurancarboxamide
2-methyl-N-[1-[(4-methoxyphenyl)methyl]-4-piperidinyl]-2-benzofurancarboxamide
7-chloro-N-[1-[(4-methoxyphenyl)methyl]-4-piperidinyl]-2-benzofurancarboxamide
5-chloro-N-[1-[(4-methoxyphenyl)methyl]-4-piperidinyl]-2-benzofurancarboxamide
6-amino-N-[1-[(4-methoxyphenyl)methyl]-4-piperidinyl] -2-benzofurancarboxamide
N-[1-[(4-methoxyphenyl)methyl]-4-piperidinyl]-5-nitro -2-benzofurancarboxamide
N-[1-[(4-methoxyphenyl)methyl]-4-piperidinyl]-6-nitro -Z-benzofurancarboxamide
N-[1-[(3-methoxyphenyl)methyl]-4-piperidinyl]-2-benzofurancarboxamide
N-[1-[(2,4-dimethoxphenyl)methyl]-4-piperidinyl]-2-benzofurancarboxamide
N-[1-[(4-N,N-dimethylaminophenyl)methyl]-4-piperidinyl]-2-benzofurancarboxamide
N-[1-[(4-methoxyphenyl)methyl]-4-piperidinyl]-6-nitro -2-benzofurancarboxamide
N-[1-[(3-methoxyphenyl)methyl]-4-piperidinyl]-2-benzofurancarboxamide
N-[1-[(2,4-dimethoxyphenyl)methyl]-4-piperidinyl]-2-benzofurancarboxamide
N-[1-[(4-N,N-dimethylaminophenyl)methyl]-4piperidinyl]-2-benzofurancarboxamide
7-methoxy-N-[1-[(4-methoxyphenyl)methyl]-4-piperidinyl]-2-benzofurancarboxamide, monohydrochloride
6,7-dimethoxy-N-[1-[(4-methoxyphenyl)methyl]-4-piperidinyl]-2-benzofurancarboxamide
N-[1-[(4-methoxyphenyl)methyl]-4-piperidinyl]-2-oxo-2H-1-benzopyran-3-carboxamide
N-[1-[(4-methoxyphenyl)methyl]-4-piperidinyl]-4-oxo-4H-1-benzopyran-3-carboxamide, monohydrochloride
N-[1-[(4-methoxyphenyl)methyl]-4-piperidinyl]-2-thiophenecarboxamide
N-[1-[(4-methoxyphenyl)methyl]-4-piperidinyl]-2-benzo[b]thiophenecarboxamide
N-[1-[[4-[(4-chlorophenyl)methoxy]phenyl]methyl]-4-peperidinyl]benzamide
N-[1-(phenylmethyl)-4-piperidinyl]-1,3-benzodioxole-5-carboxamide
N-[1-[(4-methoxyphenyl)methyl]-4-piperidinyl]-1,3-benzodioxole-5-carboxamide
2,6-dichloro-N-[1-[(4-methoxyphenyl)methyl]-4-piperidinyl]benzamide
4-(benzoylamino)-1-[(4-methoxyphenyl)methyl]-1-methylpiperidinium, iodide
7-nitro-N-[1-(phenylmethyl)-4-piperidinyl]-2-benzofurancarboxamide
7-nitro-N-[1-[(4-nitrophenyl)methyl]-4-piperidinyl]-2-benzofurancarboxamide
N-[1-[[4- (methylsulfonyl)amino]phenyl]methyl]-4-piperidinyl]-2-benzofurancarboxamide
7-[(methylsulfonyl)amino]-N-[1-(phenylmethyl)-4-piperidinyl]-2-benzofurancarboxamide
7-[(methylsulfonyl)amino]-N-[l-[[4-[(methylsulfonyl)amino]phenyl]methyl]-4-piperidinyl]-2-benzofurancarboxamide

5. Use of a compound as claimed in Claim 1 for preparing a medicament for regulating cardiac arrhythmias in mammal.

6. Use according to Claim 5 wherein said compound is selected from the group consisting of
N-[1-[(4-methoxyphenyl)methyl]-4-piperidinyl 2E-buteneamide
N-[1- 4-methoxyphenyl)methyl]-4-piperidinyl]-3-phenyl-2E-propenamide
3-phenyl-N-[1-[(propoxyphenyl)methyl]-4-piperidinyl]-2E-propenamide
3-(2-methoxyphenyl)-N-[1-[(4-methoxyphenyl)methyl]-4-piperidinyl]-2Z-propenamide
3-(2-methoxyphenyl)-N-[1-[(4-methoxyphenyl)methyl]-4-piperidinyl-2Z-propenamide, monohydrochloride
3-(4-methoxyphenyl)-N-[1-[(4-methoxyphenyl)methyl]-4-piperidinyl]-2E-propenamide
N-[1-[(4-methoxyphenyl)methyl]-4-piperidinyl]-3-(4-nitrophenyl)-2E-propenamide
N-[1-[(4-methoxyphenyl)methyl]-4-piperidinyl]-3-[4-[(methylsulfonyl)amino]phenyl]-2E-propenamide
3-(2,6-dichlorophenyl)-N-[1-[(4-methoxyphenyl)methyl] -4-piperidinyl]-2E-propenamide
3-(2-furanyl)-N-[1-[(4-methoxyphenyl)methyl]-4-piperidinyl]-2E-propenamide
3-(1H-imidazol-4-yl)-N-[(4-methoxyphenyl)methyl]-4-piperidinyl]-2E-propenamide
2-methylpropyl 4-[3-[[1-[(4-methoxyphenyl)methyl]-4-piperidinyl]amino]-3-oxo-lE-propenyl]-1H-imidazole-1-carboxylate
N-[1-[(4-methoxyphenyl)methyl]-4-piperidinyl]-3-(3-pyridinyl)-2E-propenamide
N-[1-[(4-methoxyphenyl)methyl]-4-piperidinyl]-3-phenyl-2-propynamide, monohydrochloride
N-[1-[(4-methoxyphenyl)methyl]-4-piperidinyl]-2-phenylcyclopropanecarboxamide
2,3-dihydro-N-[1-[(4-methoxyphenyl)methyl]-4-piperidinyl]-1H-indene-1-carboxamide
N-[1-[(4-methoxyphenyl)methyl]-4-piperidinyl]-3-methyl-lH-indene-2-carboxamide
1,2,3,4-tetrahydro-N-[1-[(4-methoxphenyl)methyl]-4-piperidinyl]-2-naphthalenecarboxamide
(-)1,2,3,4-tetrahydro-N-[1-[(4-methoxyphenyl)methyl]-4-piperidinyl]-2-naphthalenecarboxamide
(+)1,2,3,4-tetrahydro-N-[1-[(4-methoxyphenyl)methyl]-4-piperidinyl]-2-naphthalenecarboxamide
2,3-dihydro-N-[1-[(4-methoxyphenyl)methyl]-4-piperidinyl]-2-benzofurancarboxamide
N-(1-methyl-4-piperidinyl)-2-benzofurancarboxamide
N-[1-(cyclohexylmethyl)-4-piperidinyl]-2-benzofurancarboxamide
N-[1-(phenylmethyl)-4-piperidinyl]-2-benzofurancarboxamide
N-[1-[(4-chlorophenyl)methyl]-4-piperidinyl)-2-benzofurancarboxamide
N-[1-[(4-hydroxyphenyl)methyl]-4-piperidinyl]-2-benzofurancarboxamide
N-[1-[(4-methoxyphenyl)methyl]-4-piperidinyl]-2-benzofurancarboxamide
N-[1-[(4-methoxyphenyl)methyl]-4-piperidinyl]-3-benzofurancarboxamide
N-[1-[(4-methoxyphenyl)methyl]-3-piperidinyl-2-benzofurancarboxamide
4-[(2-benzofuranylcarbonyl)amino]-1-[(4-methoxyphenyl)methyl]-1-methylpiperidinium iodide N-[1-[(4-methoxyphenyl)methyl]-4-piperidinyl]-2-benzofurancarboxamide, N-oxide
N-[1-[(4-propoxyphenyl)methyl]-4-piperidinyl]-2-benzofurancarboxamide
N-[1-[(4-nitrophenyl)methyl]-4-piperidinyl]-2-benzofurancarboxamide
N-[1-[[4-(acetylamino)phenyl]methyl]-4-piperidinyl]-2-benzofurancarboxamide
2-methyl-N-[1-[(4-methoxyphenyl)methyl]-4-piperidinyl]-2-benzofurancarboxamide
7-chloro-N-[1-[(4-methoxyphenyl)methyl]-4-piperidinyl]-2-benzofurancarboxamide
5-chloro-N-[1-[(4-methoxyphenyl)methyl]-4-piperidinyl]-2-benzofurancarboxamide
6-amino-N-[1-[(4-methoxyphenyl)methyl]-4-piperidinyl] -2-benzofurancarboxamide
N-[1-[(4-methoxyphenyl)methyl]-4-piperidinyl]-5-nitro -2-benzofurancarboxamide
N-[1-[(4-methoxyphenyl)methyl]-4-piperidinyl]-6-nitro -Z-benzofurancarboxamide
N-[1-[(3-methoxyphenyl)methyl]-4-piperidinyl]-2-benzofurancarboxamide
N-[1-[(2,4-dimethoxphenyl)methyl]-4-piperidinyl]-2benzofurancarboxamide
N-[1-[(4-N,N-dimethylaminophenyl)methyl]-4-piperidinyl]-2-benzofurancarboxamide
N-[1-[(4-methoxyphenyl)methyl]-4-piperidinyl]-6-nitro -2-benzofurancarboxamide
N-[1-[(3-methoxyphenyl)methyl]-4-piperidinyl]-2-benzofurancarboxamide
N-[1-[(2,4-dimethoxyphenyl)methyl]-4-piperidinyl]-2-benzofurancarboxamide
N-[1-[(4-N,N-dimethylaminophenyl)methyl]-4-piperidinyl]-2-benzofurancarboxamide
7-methoxy-N-[1-[(4-methoxyphenyl)methyl]-4-piperidinyl]-2-benzofurancarboxamide, monohydrochloride
6,7-dimethoxy-N-[1-[(4-methoxyphenyl)methyl]-4-piperidinyl]-2-benzofurancarboxamide
N-[1-[(4-methoxyphenyl)methyl]-4-piperidinyl]-2-oxo-2H-1-benzopyran-3-carboxamide
N-[1-[(4-methoxyphenyl)methyl]-4-piperidinyl]-4-oxo-4H-1-benzopyran-3-carboxamide, monohydrochloride
N-[1-[(4-methoxyphenyl)methyl]-4-piperidinyl]-2-thiophenecarboxamide
N-[1-[(4-methoxyphenyl)methyl]-4-piperidinyl]-2-benzo[b]thiophenecarboxamide
N-[1-[[4-[(4-chlorophenyl)methoxy]phenyl]methyl]-4-peperidinyl]benzamide
N-[1-(phenylmethyl)-4-piperidinyl]-!,3-benzodioxole-5-carboxamide
N-[1-[(4-methoxyphenyl)methyl]-4-piperidinyl]-1,3-benzodioxole-5-carboxamide

2,6-dichloro-N-[1-[(4-methoxyphenyl)methyl]-4-piperidinyl]benzamide
4-(benzoylamino)-1-[(4-methoxyphenyl)methyl]-1-methylpiperidinium, iodide
7-nitro-N-[1-(phenylmethyl)-4-piperidinyl]-2-benzofurancarboxamide
7-nitro-N-[1-[(4-nitrophenyl)methyl]-4-piperidinyl]-2-benzofurancarboxamide
N-[1-[[4-[(methylsulfonyl)amino]phenyl]methyl]-4-piperidinyl]-2-benzofurancarboxamide
7-[(methylsulfonyl)amino]-N-[1-(phenylmethyl)-4-piperidinyl]-2-benzofurancarboxamide
7-[(methylsulfonyl)amino]-N-[1-[[4-[(methylsulfonyl)amino]phenyl]methyl]-4-piperidinyl]-2-benzofurancarboxamide

European Patent Office

# PARTIAL EUROPEAN SEARCH REPORT

which under Rule 45 of the European Patent Convention shall be considered, for the purposes of subsequent proceedings, as the European search report

Application number: EP 90 11 7085

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | JOURNAL OF MEDICINAL CHEMISTRY, vol. 17, no. 7, July 1974, pages 739-744; J.L. ARCHIBALD et al.: "Benzamidopiperidines. 3. Carbocyclic derivatives related to indoramin" | | C 07 D 211/58<br>C 07 D 211/94<br>C 07 D 401/12<br>C 07 D 405/12<br>C 07 D 409/12<br>A 61 K 31/445 |
| | * Entire publication; especially compound 15, table II * | 1-6 | |
| | -- | | |
| X,P | EP-A-0 343 307 (FABRICA ESPANOLA DE PRODUCTOS QUIMICOS Y FARMACEUTICOS) | | |
| | * Entire publication; especially compounds of examples 9,10,16 * | 1-6 | |
| | -- | | TECHNICAL FIELDS SEARCHED (Int. Cl 4) |
| X | EP-A-0 160 422 (BOC) | | |
| | * Abstract; claim 1, example V * | 1-6 | C 07 D 211/00<br>C 07 D 401/00<br>C 07 D 405/00<br>C 07 D 409/00 |
| | -- ./. | | |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-6

Claims not searched:

Reason for the limitation of the search:

See sheet -C-

| Place of search | Date of completion of the search | Examiner |
|---|---|---|
| THE HAGUE | 15-11-1990 | KISSLER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number
EP 90 11 7085
-2-

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | GB-A-1 537 867 (WYETH)<br>* Abstract; examples 1,2; claim 1 * | 1-6 | |
| | -- | | |
| A | LU-A- 78 630 (ANPHAR)<br>* Entire document; especially examples 2,3,4,5; claim 1 * | 1-6 | |
| | -- | | TECHNICAL FIELDS SEARCHED (Int Cl 4) |
| X | CH-A- 628 885 (ANPHAR)<br>* Entire document; especially claims 18,19 and examples 1-10 * | 1-6 | |
| | -- | | |
| X,P | CHEMICAL ABSTRACTS, vol. 112, 1990, page 575, abstract no. 35802w, Columbus, Ohio, US; M. KITAZAWA et al.: "Studies on the synthesis of antiulcer agents. IV. Synthesis and antiulcer activity of dihydrobenzofuranone derivatives", & YAKUGAKU ZASSHI 1989, 109(4), 232-40<br>* Entire abstract * | 1-6 | |
| | -- | | |
| X | CHEMICAL ABSTRACTS, vol. 106, 1987, page 728, abstract no. 196262m, Columbus, Ohio, US; & JP-A-62 12 757 (YOSHITOMI PHARMACEUTICAL INDUSTRIES LTD) 21-01-1987<br>* Entire abstract * | 1-6 | |
| | -- | | |
| A | FR-A-2 534 255 (DELALANDE)<br>* Abstract * | 1-6 | |
| | ---- | | |

Obscurities and contradictions

(Art. 84, EPC)

1. Claim 1 shows chemical contradictions such as "alkenyl or alkynyl of from one to 10 carbon atoms;" or "aralkenyl or aralkynyl of from one to 10 carbon atoms".
2. Claim 1, page 55, lines 7-22, lacks concise definition and it is hence impossible to search all the compounds that fall within this definition.
3. Due to the large variety of substituents and the substitution pattern on the piperidine ring a meaningful and comprehensive search is not possible.
   Only N-benzyl-4-amido piperidines have been searched.

Inconsistencies

The benzamide derivatives of claim 2 (p.64/65) and claim 4 (p.73/74) are inconsistent with the definition in claim 1 and have not been searched.

* and claim 6 (p.83)